(19) Europäisches Patentamt / European Patent Office / Office européen des brevets

(11) **EP 3 698 367 B1**

(12) **EUROPEAN PATENT SPECIFICATION**

(45) Date of publication and mention of the grant of the patent:
**14.02.2024 Bulletin 2024/07**

(21) Application number: **18797405.0**

(22) Date of filing: **17.10.2018**

(51) International Patent Classification (IPC):
**G16B 5/20** *(2019.01)*      **G16B 20/10** *(2019.01)*
**G16B 40/10** *(2019.01)*      **G16B 25/00** *(2019.01)*

(52) Cooperative Patent Classification (CPC):
**G16B 5/20; G16B 20/10; G16B 25/00; G16B 40/10**

(86) International application number:
**PCT/US2018/056297**

(87) International publication number:
**WO 2019/079458 (25.04.2019 Gazette 2019/17)**

(54) **DETECTION DEVICE AND METHOD**

DETEKTIONSVORRICHTUNG UND -VERFAHREN

DISPOSITIF ET PROCÉDÉ DE DÉTECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2017 US 201762573475 P**

(43) Date of publication of application:
**26.08.2020 Bulletin 2020/35**

(73) Proprietor: **Affymetrix, Inc.**
**Carlsbad, CA 92008 (US)**

(72) Inventors:
• **KAMNEVA, Olga**
  **Sunnyvale**
  **California 94086 (US)**
• **GOLLUB, Jeremy**
  **Carlsbad**
  **California 92008 (US)**
• **VARMA, Ram**
  **Carlsbad**
  **California 92008 (US)**

(74) Representative: **Thermo Fisher Scientific-Life Sciences Solutions Group**
**Life Technologies**
**Frankfurter Straße 129b**
**64293 Darmstadt (DE)**

(56) References cited:
WO-A2-02/093810      WO-A2-2006/010022
AU-B2- 778 358

• EMRE GÜNGÖR ET AL: "Distance and density based clustering algorithm using Gaussian kernel", EXPERT SYSTEMS WITH APPLICATIONS, vol. 69, 1 March 2017 (2017-03-01), pages 10-20, XP055545275, GB ISSN: 0957-4174, DOI: 10.1016/j.eswa.2016.10.022

**Description**

BACKGROUND

Copy-number variation (CNV) has emerged as an important type of genetic variation and is frequently incorporated into genetic analysis of genetic traits, for example, for humans. CNVs in certain genomic regions, in particular, are known to be associated with various phenotypes from rates of drug metabolism to organ transplantation outcomes. For example, the metabolism pathways for a variety of drugs are dependent on how many functional copies of a specific gene an individual organism (e.g., eukaryote and prokaryote organisms as well as animals, plants, and microorganisms) has. An individual organism with a different number of functional copies of the gene may have wildly different rates of drug metabolism when administered a standard dosage, leading to severe medical complications. That is why CNVs in predefined regions are frequently surveyed within Genome-Wide Association Studies (GWAS) performed on the basis of microarray genotyping data. However, efficient, accurate and high throughput determination of copy number states with microarray data remains difficult to achieve.

AU778358B2 discloses a process for evaluating chemical and biological assays, disclosing a method for making inferences as to the extent of random error present in replicate genomic samples composed of small numbers of data points and a method for distinguishing among different classes of probe intensities.

SUMMARY

The invention is defined by the appended claims.

In one aspect, a method for genotyping copy number variants is provided. The method may include applying density-based clustering to a data graph to generate a plurality of candidate models from Gaussian components; selecting a best-fit model from the plurality of candidate models, the selecting of the best-fit model including: applying a model from the plurality of candidate models, and a scoring function to the components, to generate a component score; selecting a plate effect value; selecting a component label for each component based on the component score; utilizing the plate effect value as a point estimate for each of the components and calculating probabilities of the component's estimated statistical parameters; the statistical parameters including mean, standard deviation and plate effect value for the components; evaluating the model's fit against a probability tolerance; evaluating a next model if the model is not within the probability tolerance; and applying the model with the highest median probability over parameters, if no one of the plurality of candidate models meets the probability tolerance; configuring a normalizer with historical component data to adjust the mean and standard deviation of each of the components to generate an adjusted mixture; configuring a classifier with the adjusted mixture to classify unknown samples, the configuring of the classifier including: weighting component densities based on the adjusted mixture; and comparing the unknown samples to the most probable components; and assigning a copy number call corresponding to the component with the highest probability to the unknown samples if the ratio of the density of the most likely component to the density of the second most likely component, evaluated at the sample position, is above a certain cutoff, and the absolute density of the most probable component, evaluated at the sample position, is above a density cutoff.

In some embodiments, the scoring function is constructed as a product of prior densities on means of mixture components, plate effect and weights of the components.

In some embodiments, the probability tolerance further includes the median of the probabilities is greater than 0.1 and no individual probability is less than 0.001.

In some embodiments, the plurality of candidate models are arranged in descending order by complexity.

In some embodiments, the data graph includes a graph having axes representing density and median log2 ratio.

In some embodiments, the median log2 ratio values include the median of log2 ratios of intensity data to a reference value, across a plurality of measurements of a genomic region.

In some embodiments, the intensity data include fluorescent intensity measures from a microarray.

In some embodiments, the microarray includes nucleic acid probes configured to hybridize to at least 10, 000 different target sequences present in the genome or the transcriptome.

In some embodiments, the microarray includes nucleic acid probes configured to hybridize to at least 20, 000 different target sequences present in the genome or the transcriptome.

In some embodiments, the microarray includes nucleic acid probes configured to hybridize to at least 10, 000 different target exon-derived sequences present in the genome or the transcriptome.

In some embodiments, the data graph includes a graph having axes representing density and any measure of central tendency.

In some embodiments, applying density-based clustering to the data graph further includes: generating a kernel density estimation from the data graph; partitioning the data graph into a plurality of regions based on density local minima; calculate the mean and standard deviation of points for each region; merging values within a first specified distance value from another region if the number of observations is below a first threshold value; removing regions outside the first specified distance value from any other region; calculating the mean, standard deviation and proportion of data points in each region; and generating a plurality of simplified candidate models including: merging values within a second specified distance value from another region; removing values outside the second specified distance value from any other region if the number of observations is below a threshold value; and calculating the mean, standard deviation and proportion of the data points.

In some embodiments, statistical parameters further include the means, standard deviations and plate effect for the components.

In some embodiments, the historical component data may be either generic, or specific to the copy number region in question.

In another aspect, a computing apparatus for genotyping copy number variants is provided. The computing apparatus may include a processor; and a memory storing instructions that, when executed by the processor, configure the apparatus to: apply density-based clustering to a data graph to generate a plurality of candidate models from Gaussian components; select a best-fit model from the plurality of candidate models, the selection of the best-fit model including: apply a model from the plurality of candidate models and a scoring function, to the components, to generate a component score; select a plate effect value; select a component label for each component based on the component score; utilize the plate effect value as a point estimate for each of the components and calculating probabilities of the component's estimated statistical parameters; the statistical parameters including mean, standard deviation and plate effect value for the components;

evaluating the model's fit against a probability tolerance; evaluating a next model if the model is not within the probability tolerance; and apply the model with the highest median probability over parameters, if no one of the plurality of candidate models meets the probability tolerance; configure a normalizer with historical component data to adjust the mean and standard deviation of each of the components to generate an adjusted mixture; configure a classifier with the adjusted mixture to classify unknown samples, the configuring of the classifier including: weight component densities based on the adjusted mixture; and compare the unknown samples to the most probable components; and assign a copy number call corresponding to the component with the highest probability to the unknown samples if the ratio of the density of the most likely component to the density of the second most likely component, evaluated at the sample position, is above a certain cutoff, and the absolute density of the most probable component, evaluated at the sample position, is above a density cutoff.

In some embodiments, the scoring function is constructed as a product of prior densities on means of mixture components, plate effect and weights of the components.

In some embodiments, the probability tolerance further includes the median of the probabilities is greater than 0.1 and no individual probability is less than 0.001.

In some embodiments, the plurality of candidate models are arranged in descending order by complexity.

In some embodiments, the data graph includes a graph having axes representing density and median log2 ratio.

In some embodiments, the median log2 ratio values include the median of log2 ratios of intensity data to a reference value, across a plurality of measurements of a genomic region.

In some embodiments, the intensity data includes fluorescent intensity measures from a microarray.

In some embodiments, the microarray includes nucleic acid probes configured to hybridize to at least 10, 000 different target sequences present in the genome or the transcriptome.

In some embodiments, the microarray includes nucleic acid probes configured to hybridize to at least 20, 000 different target sequences present in the genome or the transcriptome.

In some embodiments, the microarray includes nucleic acid probes configured to hybridize to at least 10, 000 different target exon-derived sequences present in the genome or the transcriptome.

In some embodiments, the data graph includes a graph having axes representing density and any measure of central tendency.

In some embodiments, applying density-based clustering to the data graph further includes: generating a kernel density estimation from the data graph; partitioning the data graph into a plurality of regions based on density local minima; calculating the mean and standard deviation of points for each region; merging values within a first specified distance value from another region if the number of observations is below a first threshold value; removing regions outside the first specified distance value from any other region; calculating the mean, standard deviation and proportion of data points in each region; and generating a plurality of simplified candidate models including: merging values within a second specified distance value from another region; removing values outside the second specified distance value from any other region if the number of observations is below a threshold value; and calculating the mean, standard deviation and proportion of the data points.

In some embodiments, statistical parameters further include the means, standard deviations and plate effect for the components.

In some embodiments, the historical component data may be either generic, or specific to the copy number region in question.

## BRIEF DESCRIPTION OF THE SEVERAL VIEWS OF THE DRAWINGS

To easily identify the discussion of any particular element or act, the most significant digit or digits in a reference number refer to the figure number in which that element is first introduced.

FIG. 1 illustrates a system for genotyping copy number variants 100 in accordance with one embodiment.

FIG. 2 illustrates a process for genotyping copy number variants 200.

FIG. 3 illustrates an embodiment of a subroutine for selecting a best-fit model from a plurality of candidate models 300.

FIG. 4 illustrates an embodiment of a data model 400.

Fig. 5 illustrates a data diagram 500 showing component density values and iterations in accordance with one embodiment.

FIG. 6 illustrates log 2 ratios of probe intensities in a sample vs diploid control, minus plate effect 500, in accordance with one embodiment.

FIG. 7 illustrates a data diagram 700 showing component density weights in accordance with one embodiment.

FIG. 8 illustrates a data diagram 800 showing component density weights and iterations in accordance with one embodiment.

Fig. 9 illustrates an analytical system 900 in accordance with one embodiment.

## DETAILED DESCRIPTION

Currently used algorithms require in-plate controls that are used to mitigate plate effects. However, such controls are often unavailable for variety of sample types and non-model organisms, rendering these algorithms error-prone. Further, there are a variety of known batching factors that can affect results and that are sometimes significant enough that the batching factor must be incorporated into the model (e.g., plate effect). One current way to compensate for plate effect is to calibrate to samples with a known copy number, by applying the samples to the plate and measuring the results.

The present disclosure provides novel systems and methods to analyze copy number of test samples with higher efficiency and accuracy. In some embodiments, the systems and methods do not utilize or require a control that provides a known copy number. Thus, the systems and methods provided herewith do not have to run the control sample every time of analyses (e.g., every plate). Instead, the data obtained from test samples in each plate can be analyzed and compared internally and copy number of each sample can be determined according to the analysis. With this aspect, the systems and methods can compensate for or mitigate variable factors (e.g., plate effects) with significantly higher efficiency and accuracy than other methods, e.g., that rely on control data. In addition, the systems and methods provided herein allow accounting plate effects even if a user does not analyze data for same- or different-source samples across multiple plates, which particularly helps increase throughput.

Copy number analyses in a discovery mode are often used to examine large genome regions. Embodiments of a system and method are disclosed to employ this analysis to monitor small important regions where copy number changes are expected. The region may comprise one gene, may comprise part of a gene, or in some cases may comprise a sequence or sequences larger than a gene. The system may be employed with a microarray to call a copy number, and may perform well on 5, 10, 25, 50, 60, 70, 80, 90, 100, 110, 120, 130, 140, 150, 160, 170, 180, 190, 200, 210, 220, 230, 240, 250, or more number base pairs as well as any number of base pairs between the foregoing numbers. When applied to deletions, the system may be employed with, for example, 50 probes each separated by 5 base pairs. For amplification with 5 base pair spacing, the system may utilize 100 probes. Smaller regions may be used, with an accompanying decrease in resolution. In various embodiments, any number of base pair spacing (e.g., 1, 2, 3, 4, 5, 6, 7, 8, 9, 10, 50, 100, 200, 500, 750, 1000 or more base pair spacing) is feasible with the method and system provided herein. Also, any number of probes (e.g., 10, 20, 50, 75, 100, 150, 200, 250, 500, 750, 1000, 5000, 10,000, 50,000, 100,000 or more probes per plate) can be used.

The method may be applied to one batch (one plate) at a time, utilizing multiple samples to establish a baseline and effectively normalize the signal. Batching factors are estimated for each of the plates as part of the model, and in-plate controls are not needed. The system and method compensate for technical factors that make the reference signal the current data is compared against incompatible with the current data. The method may be applied to any known batching factor, for example, plate effect. This is particularly useful when it is not possible to compensate for plate effect directly through the application of a plate reference/control.

Information may be used from multiple samples to perform the copy number evaluation, for example using an entire microtiter plate (24, 96, 384, etc). Data from the entire plate may be collected and applied to cluster the samples and make a call on that basis. For every plate of data, for every fixed region, the data is clustered based on density and unclustered outliers are temporarily removed. For each region on one given plate, an optimal Gaussian mixture is estimated using a testing ratio from all samples on that plate. For example, a median binary logarithm ratio may be applied to discover a structure within the data. Clusters are classified through comparison for CNV identification in fixed predetermined regions. For each specified region in each sample, signals are summarized across probes as median binary logarithm ratios, using a reference signal derived from diverse samples. Once a structure is established, a priori and known data about cluster locations may be applied to the components of the mixture and the components may be labeled with copy number states, with each sample assigned a call based on the probability of membership in each distribution. Outliers which were originally removed are brought back into the analysis and assigned intermediate copy number states based on prior knowledge or expectation which give the highest probability of being in each state. The intermediate copy number states are compared with known data from the prior analysis and the outliers are classified based on which of the intermediate copy number states they are most likely to be.

The following equations embody algorithms that may be implemented in logic to carry out these processing steps:

$$\mu_n = \frac{k_0}{k_0+n}\mu_0 + \frac{n}{k_0+n}\bar{y}$$

Weighted average of the prior mean and the data

$$p\left(\mu, \sigma^2 \middle| y\right) \sim N-Inv-x^2\left(\mu_n, \frac{\sigma_n^2}{k_n}; v_n, \sigma_n{}^2\right)$$

the posterior distribution if y-N ($\mu$, $\sigma^2$) and

$$\mu, \sigma^2 \sim N-Inv-x^2\left(\mu_0, \frac{\sigma_0^2}{k_0}; v_0, \sigma+\sigma^2\right)$$

$$\mu_n = \frac{k_0}{k_0+n}\mu_0 + \frac{n}{k_0+n}\bar{y}$$
$$k_n = k_0 + n$$
$$v_n = v_0 + n$$

$$v_n\sigma_n^2 = V_0\sigma_0{}^2 + (n-1)s^2 = \frac{k_0 n}{k_0+n}\left(\bar{y}-\mu_0\right)^2$$

Weighted sum of the prior variance, the sample variance and the distance between the sample and prior means.

To calculate the first, and most complex model, kernel density is obtained using observed data, the density function is determined and is differentiated to find local minima, and the local minima values are used as points to split the set of data into regions that correspond to potential mixture components. The mean and standard deviation are calculated for the points in each region. Regions with a small number of observations are iteratively merged into more populated regions. Regions beyond a threshold distance from other regions are dropped. Part of the space below each point, (for example the estimated point for copy number zero) is shrunk by a factor of the overall threshold distance divided by the threshold distance for that point. The mean, standard deviation, and proportion of data points in each region are calculated, providing the initial and the most complex candidate model.

Simpler candidate models may be iteratively constructed by merging two components closest to one another.

The means and standard deviations of the data points within the regions are recalculated and sufficiently small components are dropped, and the model is returned - it is then the simplest model.

To find the optimal number of Gaussian components, the models are iterated through from most to least complex. A scoring function is constructed as a product of prior densities on means of mixture components, plate effect and weights of the components. A set of sensible labelings are analyzed assuming the independence of all parameters. Because positions of the clusters are defined as their point estimates, the only adjustable parameter is plate effect. A maximum score value is found and used, and the plate effect value corresponding to the maximum value of the score is used as a point estimate. The component densities are weighted by adjusted mixture fractions to classify each sample. Components with estimated weights less than a certain level are assigned that weight, and all of the weights are renormalized to sum to 1. Probabilities of estimated parameters are calculated for means and standard deviations of mixture components, and plate effect is given best labeling. If at least one probability is less than, for example, 0.001, and median of them is less than, for example, 0.1, the median probability of the parameters may be recorded for a model and the process may be repeated until a satisfactory model is found. If no models are deemed satisfactory, the model with the highest median probability over parameters is chosen.

Component densities are weighted by the adjusted mixture fractions to classify each sample, and components with estimated weights less than a certain level are assigned that weight, and all of the weights are renormalized to sum to 1. For each sample, the first and second most likely components are found and density levels are compared to each other. If the most likely component is much more likely and the density is above a certain limit, the call is assigned. If one of the conditions fails then no call is assigned.

"Call" in this context refers to: a conclusion that there is a nucleotide difference vs. some reference at a given position in an individual genome or transcriptome.

"Conjugate prior" in this context refers to: In Bayesian probability theory, if the posterior distributions $p(\theta|x)$ are in the same family as the prior probability distribution $p(\theta)$, the prior and posterior are then called conjugate distributions, and the prior is called a conjugate prior for the likelihood function. For example, the Gaussian family is conjugate to itself (or self-conjugate) with respect to a Gaussian likelihood function: if the likelihood function is Gaussian, choosing a Gaussian prior over the mean will ensure that the posterior distribution is also Gaussian. This means that the Gaussian distribution is a conjugate prior for the likelihood that is also Gaussian.

"Copy number variation" in this context refers to: a phenomenon in which sections of the genome are repeated and the number of repeats in the genome varies between individuals in the human population.

"Genome-Wide Association Study" in this context refers to: an examination of a genome-wide set of genetic variants in different individuals to see if any variant is associated with a trait. This is also known as a whole genome association study (WGA study, or WGAS).

"Dirichlet distributions" in this context refers to: a multivariate generalization of the beta distribution. Dirichlet distributions are very often used as prior distributions in Bayesian statistics, and in fact the Dirichlet distribution is the conjugate prior of the categorical distribution and multinomial distribution.

"Kernel density estimation" in this context refers to: a non-parametric way to estimate the probability density function of a random variable. Kernel density estimation is a fundamental data smoothing problem where inferences about the population are made, based on a finite data sample. In some fields such as signal processing and econometrics it is also termed the Parzen-Rosenblatt window method, after Emanuel Parzen and Murray Rosenblatt, who are usually credited with independently creating it in its current form.

"Microarray" in this context refers to: a physical support on which multiple strands of DNA sequences from different genes are attached at specific locations. For example, one such support is a silicon wafer embedded in a plastic case. Millions of DNA fragments, representing a portion of the human genome, are synthesized onto this wafer; an organism's DNA is then loaded onto the microarray to be analyzed. Other types of supports are nylon membranes and glass microscope slides, and the DNA fragments - also called probes - can be synthesized, printed, or spotted onto these supports.

"Normal-inverse-gamma distribution" in this context refers to: In probability theory and statistics, the normal-inverse-gamma distribution (or Gaussian-inverse-gamma distribution) is a four-parameter family of multivariate continuous probability distributions. It is the conjugate prior of a normal distribution with unknown mean and variance. Bayesian consistency has become closely associated with Bayesian non-parametrics, reflecting the realistic assumption that the true distribution function can take any shape. On the other hand, parametric Bayesian inference is based on prior probability 1 being put on density functions having a particular form.

"Posterior", in this context, refers to: after taking into account the relevant evidence related to the particular case being examined.

"Posterior probability" in this context refers to: the conditional probability of a random event or an uncertain proposition that is assigned after the relevant evidence or background is taken into account. Similarly, the posterior probability distribution is the probability distribution of an unknown quantity, treated as a random variable, conditional on the evidence obtained from an experiment or survey.

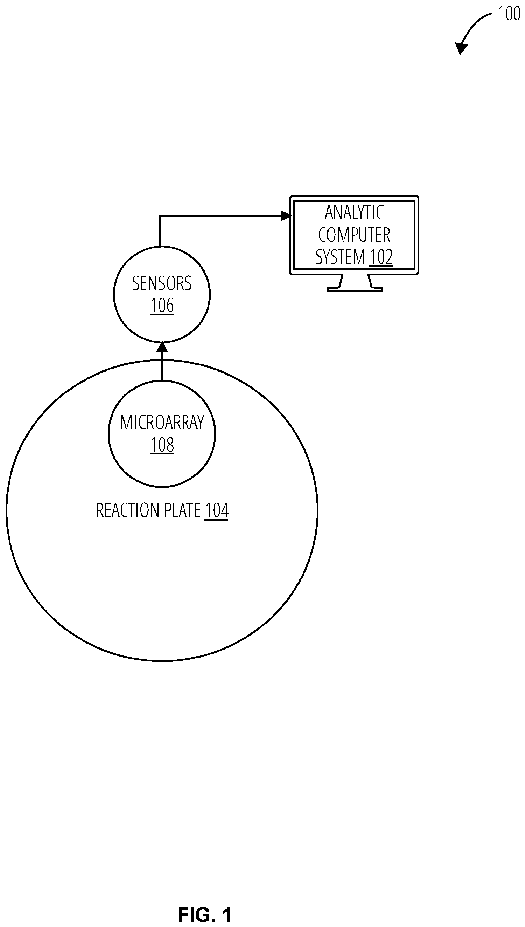

In some embodiments, the system for genotyping copy number variants 100 as illustrated in Fig. 1 comprises an analytic computer system 102, a reaction plate 104, a sensors 106, and a microarray 108.

In some embodiments, the analytic computer system 102 receives data from the sensors 106. The sensors 106 may be optical sensors to detect fluorescence from the microarray 108 on the reaction plate 104.

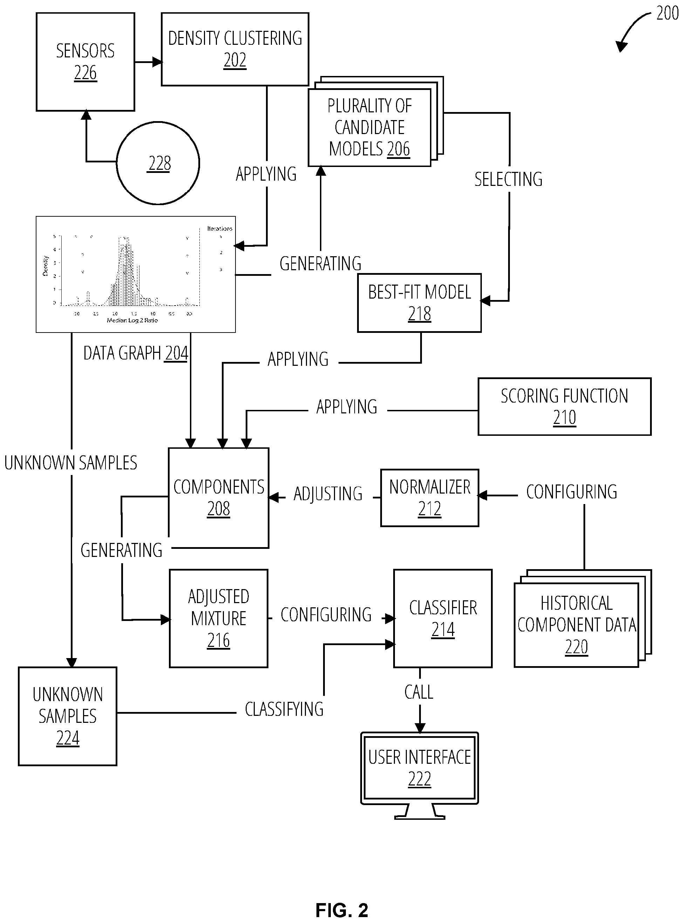

In some embodiments, the system for genotyping copy number variants 200 as illustrated Fig. 2 comprises a density clustering 202, a data graph 204, a plurality of candidate models 206, a components 208, a scoring function 210, a normalizer 212, a classifier 214, an adjusted mixture 216, a best-fit model 218, a historical component data 220, a user interface 222, an unknown samples 224, a sensors 226, and a microarray 228.

In some embodiments, the density clustering 202 is applied to the data graph 204 to generate a plurality of candidate models 206 and a best-fit model 218 is selected from the plurality of candidate models 206. The density clustering 202 may be receive data for the data graph 204 from the sensors 226 which may read fluorescence from the microarray 228. The best-fit model 218 and the scoring function 210 are applied to the components 208 from the data graph 204.

In some embodiments, the normalizer 212 is configured with the historical component data 220 to adjusted the means and standard deviations of each of the components 208 and generate an adjusted mixture 216. The classifier 214 may be configured with the adjusted mixture 216 to classify the unknown samples 224 and transmit a call to the user interface 222.

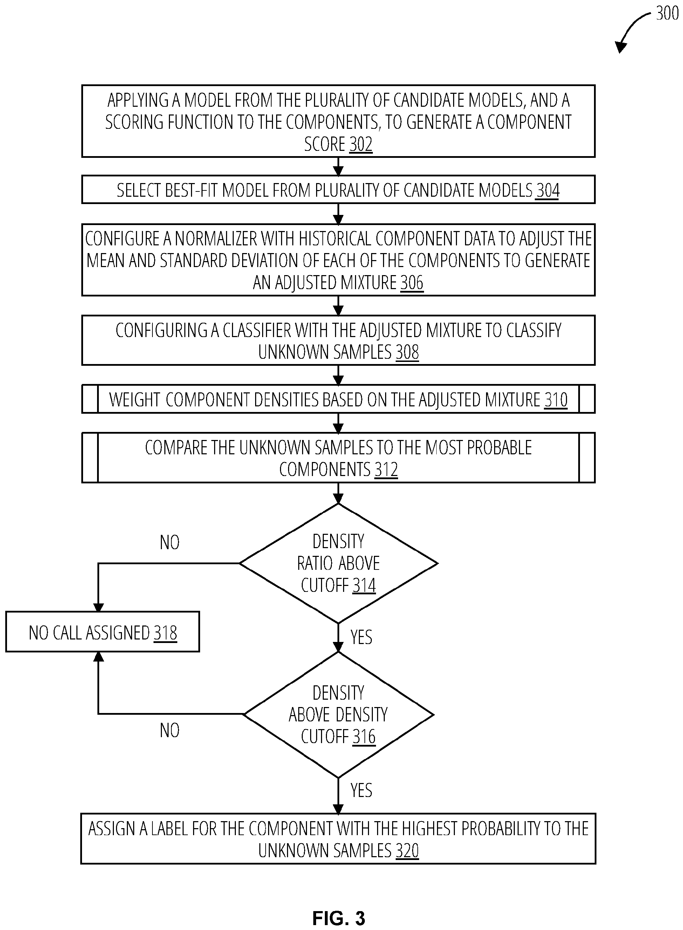

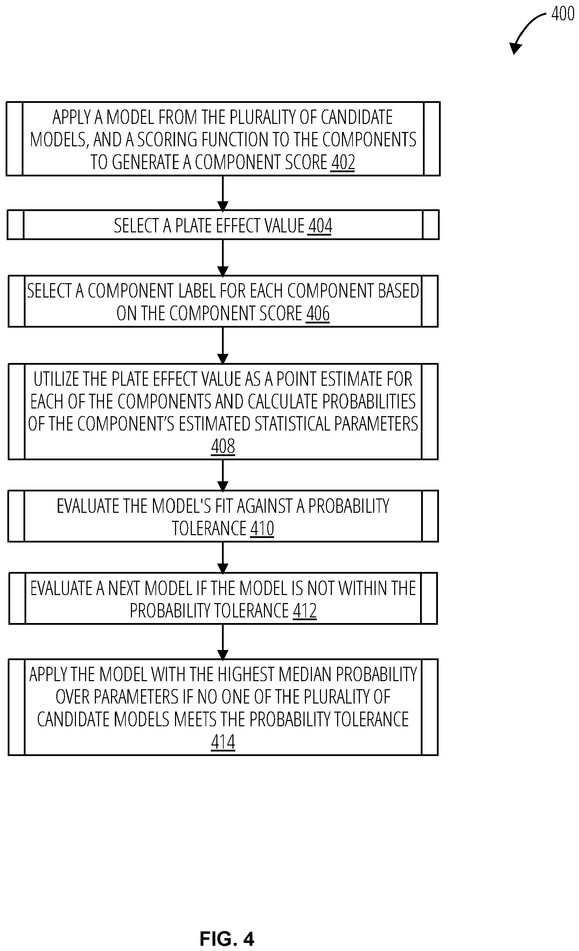

The system for genotyping copy number variants 200 may be operated in accordance with the processes outlined in Figure 3 and Figure 4. In some embodiments, one or more additional steps can be performed before, after or between any steps illustrated in various embodiments of figures herein. Thus, for example, steps of selecting samples and adding samples to an array can be performed prior to step 302 of Fig. 3. In addition, one or more steps shown in the exemplary embodiments of the figures can be omitted to the extent that would not substantially reduce the functionality of the assay.

Referencing Figure 3, a process for genotyping copy number variants 300 according to some embodiments applies density-based clustering to a data graph to generate a plurality of candidate models from Gaussian components (block 302).

In some embodiments, the process for genotyping copy number variants 300 selects a best-fit model from the plurality of candidate models (block 304).

In some embodiments, the process for genotyping copy number variants 300 configures a normalizer with historical component data to adjust the mean and standard deviation of each of the components to generate an adjusted mixture (block 306).

In some embodiments, the process for genotyping copy number variants 300 configures a classifier with the adjusted mixture to classify unknown samples (block 308).

In some embodiments, the process for genotyping copy number variants 300 weights component densities based on the adjusted mixture (subroutine block 310).

In some embodiments, the process for genotyping copy number variants 300 compares the unknown samples to the most probable components (subroutine block 312).

In some embodiments, the process for genotyping copy number variants 300 evaluates whether the ratio of the density of the most likely component to the density of the second most likely component, evaluated at the sample position, is above a certain cutoff (decision block 314).

In some embodiments, the process for genotyping copy number variants 300 evaluates whether the absolute density of the most probable component, evaluated at the sample position, is above a density cutoff (decision block 316).

In some embodiments, the process for genotyping copy number variants 300 assigns no call if either the ratio of the density of the most likely component to the density of the second most likely component, evaluated at the sample position, is below a certain cutoff, or the absolute density of the most probable component, evaluated at the sample position, is below a density cutoff (block 318).

In some embodiments, the process for genotyping copy number variants 300 assigns a label for the component with the highest probability to the unknown samples if the ratio of the density of the most likely component to the density of the second most likely component, evaluated at the sample position, is above a certain cutoff, and the absolute density of the most probable component, evaluated at the sample position, is above a density cutoff (block 320).

Referencing Figure 4, a subroutine for selecting a best-fit model from a plurality of candidate models 400 according to some embodiments applies a model from the plurality of candidate models, and a scoring function to the components, to generate a component score (subroutine block 402).

In some embodiments, the process for genotyping copy number variants 400 selects a plate effect value (subroutine block 404).

In some embodiments, the process for genotyping copy number variants 400 selects a component for each component based on the component score (subroutine block 406).

In some embodiments, the process for genotyping copy number variants 400 utilizes the plate effect value as a point estimate for each of the components and calculates the probabilities of the component's estimated statistical parameters (subroutine block 408).

In some embodiments, the process for genotyping copy number variants 400 evaluates the model's fit against a probability tolerance (subroutine block 410).

In some embodiments, the process for genotyping copy number variants 400 evaluating a next model if the model is not within the probability tolerance (subroutine block 412).

In some embodiments, the process for genotyping copy number variants 400 applies the model with the highest median probability over parameters if no one of the plurality of candidate models meets the probability tolerance (subroutine block 414).

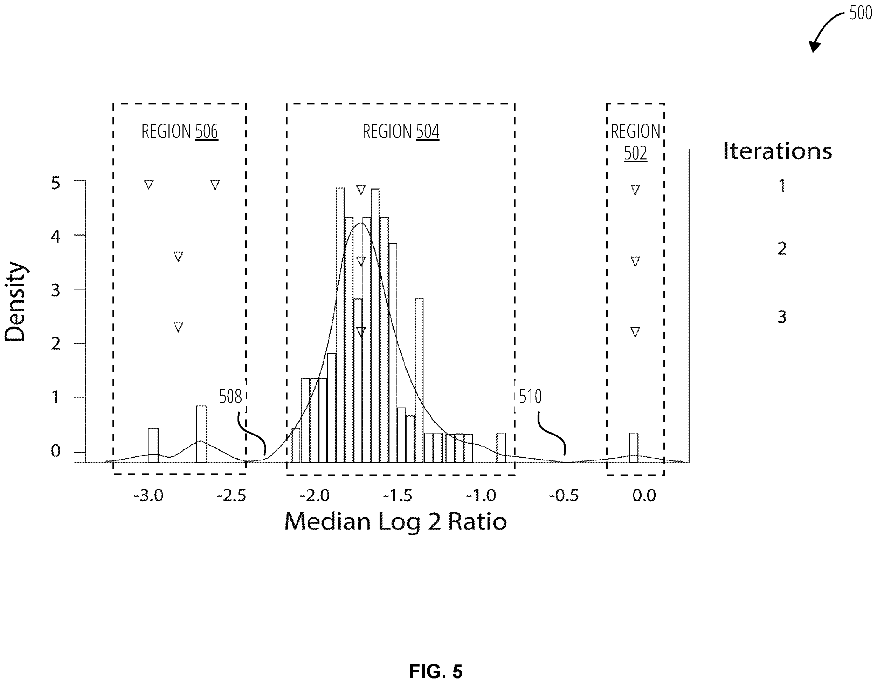

In some embodiments, the data model 500 as illustrated in Fig. 5 comprises a region 502, a region 504, a region 506, a local minima 508, and a local minima 510.

In some embodiments, Kernel density may be found using observed data. Local minima 508 and the local minima 510 may be found through differentiation of the density. The local minima 508 and the local minima 510 may be used to split the entire set of data into the region 506 the region 404 and the region 502 which correspond to potential mixture components, then calculating the mean and SD of points in each region.

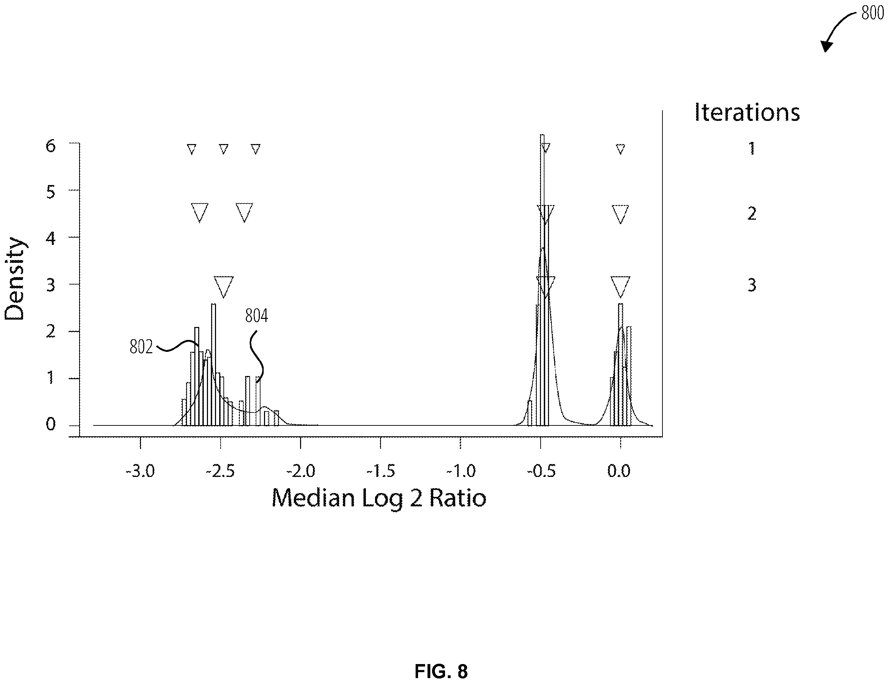

The regions may be iteratively merged into regions with larger numbers of observations. The mean, standard deviation and proportion of data points in each region may be recalculated to create the initial and the most complex candidate model. As an alternative example, a set of samples was analyzed according to the foregoing process and the resulting data is illustrated in Fig. 8 with component density weights and iterations.

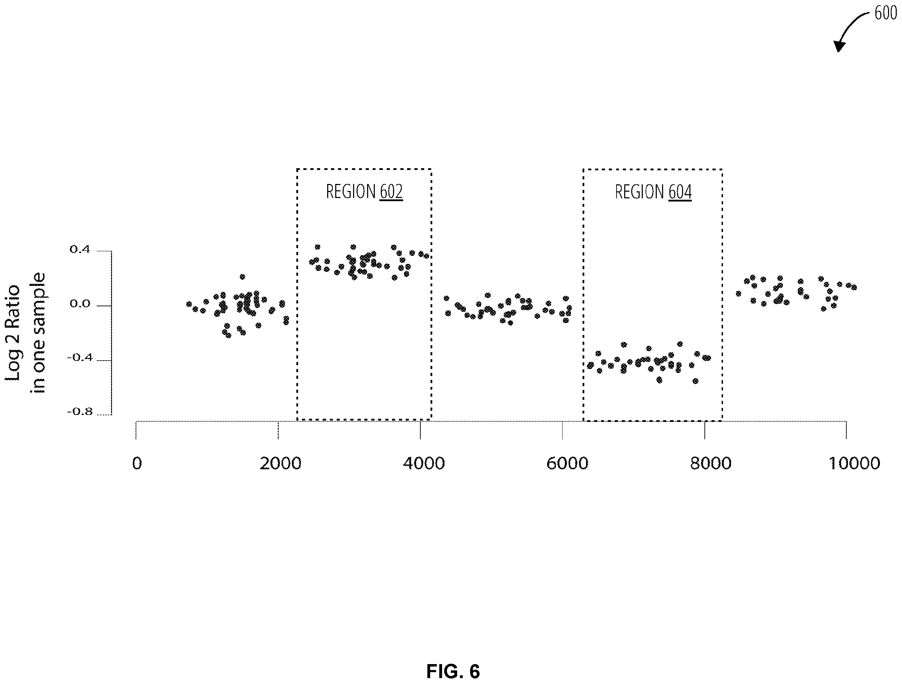

In some embodiments, the log 2 ratios of probe intensities in sample vs diploid control minus plate effect 600 as illustrated in Fig. 6 comprises a region 602 and a region 604.Fixed thresholds to determine copy number state, may be adjusted per region 602 and region 604, and per array type.

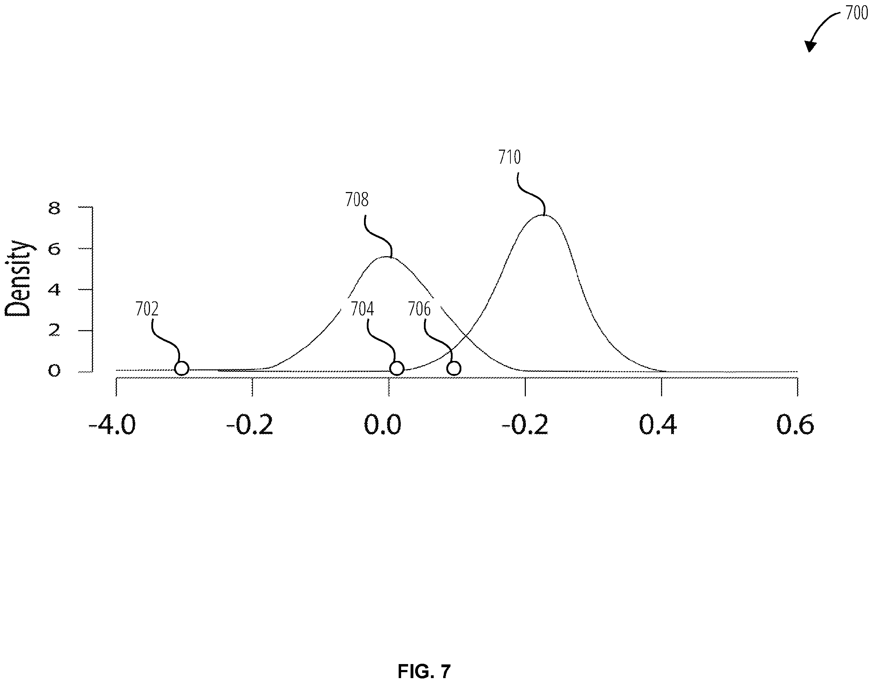

In some embodiments, the component density weights 700 as illustrated in Fig. 7 comprise a sample 702, a sample 704, a sample 706, a component density 708, and a component density 710.

The component density weighted by adjusted mixture fractions may be used to classify each of the sample 702, the sample 704, and the sample 706. Components with estimated weights less than a certain cutoff may be assigned that weight and all of the weights may be renormalized to sum to 1.

In some embodiments, for the sample 704, the sample 706 and the sample 702, the most and second most likely components, the component density 710 and the component density 708, can be found. The component density 710 and the component density 708 may be compared to each other. A call corresponding to the component density 708 may be assigned to the sample 704 based on the ratio of the component density 710 to the component density 608 and the absolute density of the component density 708. The sample 706 may be assigned to the component density 708 or the component density 710 depending on the ratio of the component density 710 to the component density 708 and the absolute densities of the component density 608 and the component density 710. No call may be assigned to the sample 702 due to its distance to the component density 710 and the component density 708, depicting a lack of absolute and relative component density.

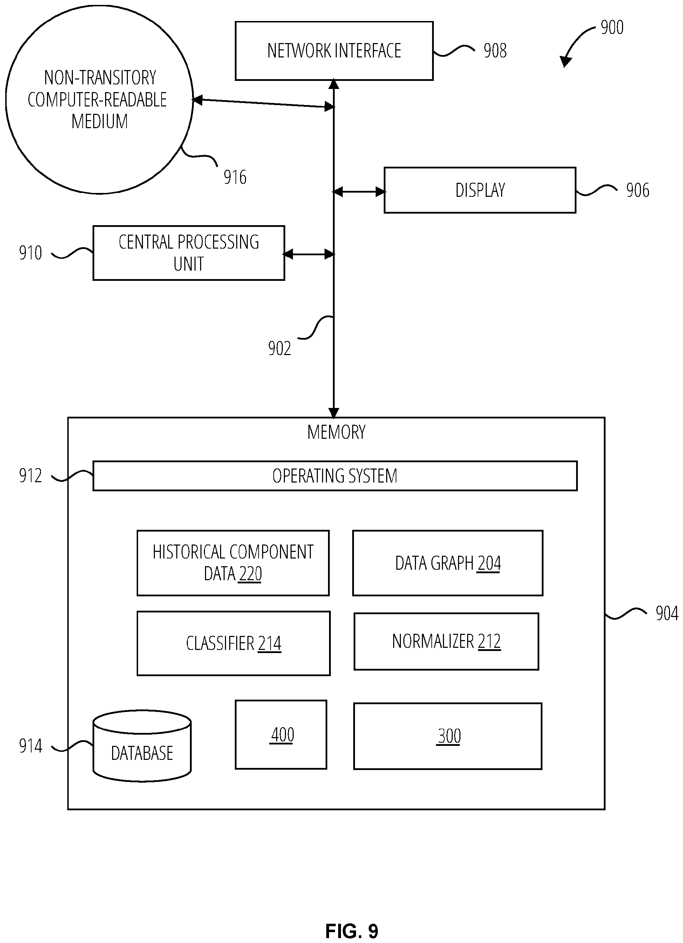

Referring to Figure 9, a system for genotyping copy number variants 900 according to some embodiments comprises a component 902 and a component 904. Figure 9 illustrates that simpler models may be constructed in an iterative fashion.

The component 902 and the component 904 may be grouped together in some embodiments, by merging with the components closes to each of them within a probability tolerance value, if they are close enough to each other. The means and standard deviations of the data point are recalculated.

Figure 9 illustrates several components of an exemplary analytical system 900 in accordance with one embodiment. In various embodiments, system 900 may include a desktop PC, server, workstation, mobile phone, laptop, tablet, set-top box, appliance, or other computing device that is capable of performing operations such as those described herein. In some embodiments, system 900 may include many more components than those shown in Figure 9. However, it is not necessary that all of these generally conventional components be shown in order to disclose an illustrative embodiment. Collectively, the various tangible components or a subset of the tangible components may be referred to herein as "logic" configured or adapted in a particular way, for example as logic configured or adapted with particular software or firmware.

In various embodiments, system 900 may comprise one or more physical and/or logical devices that collectively provide the functionalities described herein. In some embodiments, system 900 may comprise one or more replicated and/or distributed physical or logical devices.

In some embodiments, system 900 may comprise one or more computing resources provisioned from a "cloud computing" provider, for example, Amazon Elastic Compute Cloud ("Amazon EC2"), provided by Amazon.com, Inc. of Seattle, Washington; Sun Cloud Compute Utility, provided by Sun Microsystems, Inc. of Santa Clara, California; Windows Azure, provided by Microsoft Corporation of Redmond, Washington, and the like.

System 900 includes a bus 902 interconnecting several components including a network interface 908, a display 906, a central processing unit 910, and a memory 904.

Memory 904 generally comprises a random access memory ("RAM") and permanent non-transitory mass storage device, such as a hard disk drive or solid-state drive. Memory 904 stores an operating system 912.

These and other software components may be loaded into memory 904 of system 900 using a drive mechanism (not shown) associated with a non-transitory computer-readable medium 916, such as a DVD/CD-ROM drive, memory card, network download, or the like.

Memory 904 also includes database 914. In some embodiments, system 900 may communicate with database 914 via network interface 908, a storage area network ("SAN"), a high-speed serial bus, and/or via the other suitable communication technology.

In some embodiments, database 914 may comprise one or more storage resources provisioned from a "cloud storage" provider, for example, Amazon Simple Storage Service ("Amazon S3"), provided by Amazon.com, Inc. of Seattle, Washington, Google Cloud Storage, provided by Google, Inc. of Mountain View, California, and the like.

Terms used herein should be accorded their ordinary meaning in the relevant arts, or the meaning indicated by their use in context, but if an express definition is provided, that meaning controls.

"Circuitry" in this context refers to electrical circuitry having at least one discrete electrical circuit, electrical circuitry having at least one integrated circuit, electrical circuitry having at least one application specific integrated circuit, circuitry forming a general purpose computing device configured by a computer program (e.g., a general purpose computer configured by a computer program which at least partially carries out processes or devices described herein, or a microprocessor configured by a computer program which at least partially carries out processes or devices described herein), circuitry forming a memory device (e.g., forms of random access memory), or circuitry forming a communications device (e.g., a modem, communications switch, or optical-electrical equipment).

"Firmware" in this context refers to software logic embodied as processor-executable instructions stored in read-only memories or media.

"Hardware" in this context refers to logic embodied as analog or digital circuitry.

"Logic" in this context refers to machine memory circuits, non-transitory machine readable media, and/or circuitry which by way of its material and/or material-energy configuration comprises control and/or procedural signals, and/or settings and values (such as resistance, impedance, capacitance, inductance, current/voltage ratings, etc.), that may be applied to influence the operation of a device. Magnetic media, electronic circuits, electrical and optical memory (both volatile and nonvolatile), and firmware are examples of logic. Logic specifically excludes pure signals or software per se (however does not exclude machine memories comprising software and thereby forming configurations of matter).

"Programmable device" in this context refers to an integrated circuit designed to be configured and/or reconfigured after manufacturing. The term "programmable processor" is another name for a programmable device herein. Programmable devices may include programmable processors, such as field programmable gate arrays (FPGAs), configurable hardware logic (CHL), and/or any other type programmable devices. Configuration of the programmable device is generally specified using a computer code or data such as a hardware description language (HDL), such as for example Verilog, VHDL, or the like. A programmable device may include an array of programmable logic blocks and a hierarchy of reconfigurable interconnects that allow the programmable logic blocks to be coupled to each other according to the descriptions in the HDL code. Each of the programmable logic blocks may be configured to perform complex combinational functions, or merely simple logic gates, such as AND, and XOR logic blocks. In most FPGAs, logic blocks also include memory elements, which may be simple latches, flip-flops, hereinafter also referred to as "flops," or more complex blocks of memory. Depending on the length of the interconnections between different logic blocks, signals may arrive at input terminals of the logic blocks at different times.

"Software" in this context refers to logic implemented as processor-executable instructions in a machine memory (e.g. read/write volatile or nonvolatile memory or media).

Herein, references to "one embodiment" or "an embodiment" do not necessarily refer to the same embodiment, although they may. Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively, unless expressly limited to a single one or multiple ones. Additionally, the words "herein," "above," "below" and words of similar import, when used in this application, refer to this application as a whole and not to any particular portions of this application. When the claims use the word "or" in reference to a list of two or more items, that word covers all of the following interpretations of the word: any of the items in the list, all of the items in the list and any combination of the items in the list, unless expressly limited to one or the other. Any terms not expressly defined herein have their conventional meaning as commonly understood by those having skill in the relevant art(s).

Various logic functional operations described herein may be implemented in logic that is referred to using a noun or noun phrase reflecting said operation or function. For example, an association operation may be carried out by an "associator" or "correlator". Likewise, switching may be carried out by a "switch", selection by a "selector", and so on.

Those skilled in the art will recognize that it is common within the art to describe devices or processes in the fashion set forth herein, and thereafter use standard engineering practices to integrate such described devices or processes into larger systems. At least a portion of the devices or processes described herein can be integrated into a network processing system via a reasonable amount of experimentation. Various embodiments are described herein and presented by way of example and not limitation.

Those having skill in the art will appreciate that there are various logic implementations by which processes and/or systems described herein can be effected (e.g., hardware, software, or firmware), and that the preferred vehicle will vary with the context in which the processes are deployed. If an implementer determines that speed and accuracy are paramount, the implementer may opt for a hardware or firmware implementation; alternatively, if flexibility is paramount, the implementer may opt for a solely software implementation; or, yet again alternatively, the implementer may opt for some combination of hardware, software, or firmware. Hence, there are numerous possible implementations by which the processes described herein may be effected, none of which is inherently superior to the other in that any vehicle to be utilized is a choice dependent upon the context in which the implementation will be deployed and the specific concerns (e.g., speed, flexibility, or predictability) of the implementer, any of which may vary. Those skilled in the art will recognize that optical aspects of implementations may involve optically-oriented hardware, software, and or firmware.

Those skilled in the art will appreciate that logic may be distributed throughout one or more devices, and/or may be comprised of combinations memory, media, processing circuits and controllers, other circuits, and so on. Therefore, in the interest of clarity and correctness logic may not always be distinctly illustrated in drawings of devices and systems, although it is inherently present therein. The techniques and procedures described herein may be implemented via logic distributed in one or more computing devices. The particular distribution and choice of logic will vary according to implementation.

The foregoing detailed description has set forth various embodiments of the devices or processes via the use of block diagrams, flowcharts, or examples. Insofar as such block diagrams, flowcharts, or examples contain one or more functions or operations, it will be understood as notorious by those within the art that each function or operation within such block diagrams, flowcharts, or examples can be implemented, individually or collectively, by a wide range of hardware, software, firmware, or virtually any combination thereof. Portions of the subject matter described herein may be implemented via Application Specific Integrated Circuits (ASICs), Field Programmable Gate Arrays (FPGAs), digital signal processors (DSPs), or other integrated formats. However, those skilled in the art will recognize that some aspects of the embodiments disclosed herein, in whole or in part, can be equivalently implemented in standard integrated circuits, as one or more computer programs running on one or more processing devices (e.g., as one or more programs running on one or more computer systems), as one or more programs running on one or more processors (e.g., as one or more programs running on one or more microprocessors), as firmware, or as virtually any combination thereof, and that designing the circuitry or writing the code for the software or firmware would be well within the skill of one of skill in the art in light of this disclosure. In addition, those skilled in the art will appreciate that the mechanisms of the subject matter described herein are capable of being distributed as a program product in a variety of forms, and that an illustrative embodiment of the subject matter described herein applies equally regardless of the particular type of signal bearing media used to actually carry out the distribution. Examples of a signal bearing media include, but are not limited to, the following: recordable type media such as floppy disks, hard disk drives, CD ROMs, digital tape, flash drives, SD cards, solid state fixed or removable storage, and computer memory.

**Claims**

1. A computer-implemented method for genotyping copy number variants comprising:

    receiving fluorescence data for a data graph from a microarray including nucleic acid probes;
    applying density-based clustering to the data graph to generate a plurality of candidate models from Gaussian components;
    selecting a best-fit model from the plurality of candidate models, the selecting of the best-fit model comprising:

        applying a model from the plurality of candidate models, and a scoring function to the components, to generate a component score;
        selecting a plate effect value;
        selecting a component label for each component based on the component score;
        utilizing the plate effect value as a point estimate for each of the components and calculating probabilities of the component's estimated statistical parameters, the statistical parameters including mean, standard deviation and plate effect value for the components;

evaluating the model's fit against a probability tolerance;
evaluating a next model if the model is not within the probability tolerance; and
applying the model with the highest median probability over parameters, if no one of the plurality of candidate models meets the probability tolerance;

configuring a normalizer with historical component data to adjust the mean and standard deviation of each of the components to generate an adjusted mixture;
configuring a classifier with the adjusted mixture to classify unknown samples, the configuring of the classifier comprising:

weighting component densities based on the adjusted mixture; and
comparing the unknown samples to the most probable components; and

assigning a copy number call corresponding to the component with the highest probability to the unknown samples if the ratio of the density of the most likely component to the density of the second most likely component, evaluated at the sample position, is above a certain cutoff, and the absolute density of the most probable component, evaluated at the sample position, is above a density cutoff.

2. The method of claim 1 wherein the scoring function is constructed as a product of prior densities on means of mixture components, plate effect and weights of the components.

3. The method of any one of claims 1-2 wherein the probability tolerance further comprises the median of the probabilities is greater than 0.1 and no individual probability is less than 0.001.

4. The method of any one of claims 1-3 wherein the data graph comprises a graph having axes representing density and median log2 ratio.

5. The method of any one of claims 1-4, wherein the median log2 ratio values comprise the median of log2 ratios of intensity data to a reference value, across a plurality of measurements of a genomic region.

6. The method of claim 5, wherein the intensity data include fluorescent intensity measures from a microarray.

7. The method of any one of claims 1-6 wherein the data graph comprises a graph having axes representing density and any measure of central tendency.

8. The method of any one of claims 1-7 wherein applying density-based clustering to the data graph further comprises:

generating a kernel density estimation from the data graph;
partitioning the data graph into a plurality of regions based on density local minima;
calculate the mean and standard deviation of points for each region;
merging values within a first specified distance value from another region if the number of observations is below a first threshold value;
removing regions outside the first specified distance value from any other region;
calculating the mean, standard deviation and proportion of data points in each region; and
generating a plurality of simplified candidate models comprising:

merging values within a second specified distance value from another region;
removing values outside the second specified distance value from any other region if the number of observations is below a threshold value; and
calculating the mean, standard deviation and proportion of the data points.

9. A computing apparatus for genotyping copy number variants, the computing apparatus comprising:

a processor; and
a memory storing instructions that, when executed by the processor, configure the apparatus to:

receive fluorescence data for a data graph from a microarray including nucleic acid probes;
apply density-based clustering to the data graph to generate a plurality of candidate models from Gaussian components;
select a best-fit model from the plurality of candidate models, the selection of the best-fit model comprising:

apply a model from the plurality of candidate models and a scoring function, to the components, to generate a component score;
select a plate effect value;
select a component label for each component based on the component score;
utilize the plate effect value as a point estimate for each of the components and calculating probabilities of the component's estimated statistical parameters, the statistical parameters including mean, standard deviation and plate effect value for the components;
evaluating the model's fit against a probability tolerance;
evaluating a next model if the model is not within the probability tolerance; and
apply the model with the highest median probability over parameters, if no one of the plurality of candidate models meets the probability tolerance;

configure a normalizer with historical component data to adjust the mean and standard deviation of each of the components to generate an adjusted mixture;
configure a classifier with the adjusted mixture to classify unknown samples, the configuring of the classifier comprising:

weight component densities based on the adjusted mixture; and
compare the unknown samples to the most probable components; and

assign a copy number call corresponding to the component with the highest probability to the unknown samples if the ratio of the density of the most likely component to the density of the second most likely component, evaluated at the sample position, is above a certain cutoff, and the absolute density of the most probable component, evaluated at the sample position, is above a density cutoff.

10. The computing apparatus of claim 9 wherein the scoring function is constructed as a product of prior densities on means of mixture components, plate effect and weights of the components.

11. The computing apparatus of any one of claims 9-10 wherein the probability tolerance further comprises the median of the probabilities is greater than 0.1 and no individual probability is less than 0.001.

12. The computing apparatus of any one of claims 9-11 wherein the data graph comprises a graph having axes representing density and median log2 ratio.

13. The computing apparatus of claim 12, wherein the median log2 ratio values comprise the median of log2 ratios of intensity data to a reference value, across a plurality of measurements of a genomic region.

14. The computing apparatus of any one of claims 9-13 wherein the data graph comprises a graph having axes representing density and any measure of central tendency.

15. The computing apparatus of any one of claims 9-14 wherein applying density-based clustering to the data graph further comprises:

generating a kernel density estimation from the data graph;
partitioning the data graph into a plurality of regions based on density local minima;
calculating the mean and standard deviation of points for each region;
merging values within a first specified distance value from another region if the number of observations is below a first threshold value;
removing regions outside the first specified distance value from any other region;
calculating the mean, standard deviation and proportion of data points in each region; and
generating a plurality of simplified candidate models comprising:

merging values within a second specified distance value from another region;
removing values outside the second specified distance value from any other region if the number of observations is below a threshold value; and

calculating the mean, standard deviation and proportion of the data points.

**Patentansprüche**

1. Computerimplementiertes Verfahren zum Genotypisieren von Kopienzahlvarianten, umfassend:

    Empfangen von Fluoreszenzdaten für ein Datendiagramm von einem Mikroarray, das Nucleinsäureproben enthält;
    Anwenden eines dichtebasierten Clusterings auf das Datendiagramm, um eine Vielzahl von Kandidatenmodellen aus Gaußschen Komponenten zu erzeugen;
    Auswählen eines am besten passenden Modells aus der Vielzahl von Kandidatenmodellen, das Auswählen des am besten passenden Modells umfassend:

        Anwenden eines Modells aus der Vielzahl von Kandidatenmodellen und einer Bewertungsfunktion auf die Komponenten, um eine Komponentenbewertung zu erzeugen;
        Auswählen eines Platteneffektwerts;
        Auswählen einer Komponentenbezeichnung für jede Komponente basierend auf der Komponentenbewertung;
        Nutzen des Platteneffektwerts als eine Punktschätzung für jede der Komponenten und Berechnen von Wahrscheinlichkeiten der geschätzten statistischen Parameter, wobei die statistischen Parameter einen Mittelwert, eine Standardabweichung und Platteneffektwert für die Komponenten einschließen;
        Auswerten der Passung des Modells gegen eine Wahrscheinlichkeitstoleranz;
        Auswerten einer Passung des nächsten Modells, falls das Modell nicht innerhalb der Wahrscheinlichkeitstoleranz ist; und
        Anwenden des Modells mit der höchsten medianen Wahrscheinlichkeit über Parameter, falls keines der Vielzahl von Kandidatenmodellen die Wahrscheinlichkeitstoleranz erfüllt;
        Konfigurieren eines Normalisators mit historischen Komponentendaten, um den Mittelwert und die Standardabweichung jeder der Komponenten einzustellen, um eine angepasste Mischung zu erzeugen;
        Konfigurieren eines Klassifikators mit der angepasste Mischung, um unbekannte Abtastwerte zu klassifizieren, das Konfigurieren des Klassifikators umfassend:

            Gewichten von Komponentendichten basierend auf der angepassten Mischung; und
            Vergleichen der unbekannten Abtastwerte mit den wahrscheinlichsten Komponenten; und
            Zuweisen eines Kopienzahlwertes, der der Komponente mit der höchsten Wahrscheinlichkeit entspricht, an die unbekannten Abtastwerte, falls das Verhältnis der Dichte der wahrscheinlichsten Komponente zu der Dichte der zweitwahrscheinlichsten Komponente, die an der Abtastwertposition ausgewertet wurden, über einer bestimmten Höchstgrenze liegt, und die absolute Dichte der wahrscheinlichsten Komponente, die an der Abtastwertposition ausgewertet wurde, über einer Dichtehöchstgrenze liegt.

2. Verfahren nach Anspruch 1, wobei die Bewertungsfunktion als ein Produkt von vorherigen Dichten auf Mittelwerten von Mischungskomponenten, Platteneffekt und Gewichtungen der Komponenten aufgebaut ist.

3. Verfahren nach einem der Ansprüche 1 bis 2, wobei die Wahrscheinlichkeitstoleranz ferner umfasst, dass der Median der Wahrscheinlichkeiten größer als 0,1 ist und keine individuelle Wahrscheinlichkeit weniger als 0,001 beträgt.

4. Verfahren nach einem der Ansprüche 1 bis 3, wobei das Datendiagramm ein Diagramm umfasst, das Achsen aufweist, die die Dichte und das mediane log2-Verhältnis darstellen.

5. Verfahren nach einem der Ansprüche 1 bis 4, wobei die medianen log2-Verhältniswerte den Median von log2-Verhältnissen von Intensitätsdaten zu einem Referenzwert über eine Vielzahl von Messungen einer genomischen Region hinweg umfassen.

6. Verfahren nach Anspruch 5, wobei die Intensitätsdaten Fluoreszenzintensitätsmessungen von einem Mikroarray einschließen.

7. Verfahren nach einem der Ansprüche 1 bis 6, wobei das Datendiagramm ein Diagramm umfasst, das Achsen aufweist, die die Dichte und eine beliebige Messung der zentralen Tendenz darstellen.

8. Verfahren nach einem der Ansprüche 1 bis 7, wobei das Anwenden des dichtebasierten Clusterings an das Datendiagramm ferner umfasst:

Erzeugen einer Kerneldichteschätzung aus dem Datendiagramm;
Partitionieren des Datendiagramms in eine Vielzahl von Regionen basierend auf der Dichte lokaler Minima;
Berechnen des Mittelwerts und der Standardabweichung der Punkte für jede Region;
Zusammenführen von Werten innerhalb eines ersten spezifizierten Abstandswerts von einer anderen Region, falls die Anzahl von Beobachtungen unter einem ersten Schwellenwert ist;
Entfernen von Regionen außerhalb des ersten spezifizierten Abstandswerts von einer beliebigen anderen Region;
Berechnen des Mittelwerts, der Standardabweichung und einer Proportion von Datenpunkten in jeder Region; und
Erzeugen einer Vielzahl von vereinfachten Kandidatenmodellen, umfassend:

Zusammenführen von Werten innerhalb eines zweiten spezifizierten Abstandswerts von einer anderen Region;
Entfernen von Werten außerhalb des zweiten spezifizierten Abstandswerts von einer beliebigen anderen Region, falls die Anzahl der Beobachtungen unter einem Schwellenwert ist; und
Berechnen des Mittelwerts, der Standardabweichung und der Proportion der Datenpunkte.

9. Recheneinrichtung zum Genotypisieren von Kopienzahlvarianten, die Recheneinrichtung umfassend:

einen Prozessor; und
einen Speicher, der Anweisungen speichert, die, wenn sie durch den Prozessor ausgeführt werden, die Einrichtung konfigurieren zum:

Empfangen von Fluoreszenzdaten für ein Datendiagramm von einem Mikroarray, das Nucleinsäureproben enthält;
Anwenden des dichtebasierten Clusterings auf das Datendiagramm, um eine Vielzahl von Kandidatenmodellen aus Gaußschen Komponenten zu erzeugen;
Auswählen eines am besten passenden Modells aus der Vielzahl von Kandidatenmodellen, die Auswahl des am besten passenden Modells umfassend:

Anwenden eines Modells aus der Vielzahl von Kandidatenmodellen und einer Bewertungsfunktion auf die Komponenten, um eine Komponentenbewertung zu erzeugen;
Auswählen eines Platteneffektwerts;
Auswählen einer Komponentenbezeichnung für jede Komponente basierend auf der Komponentenbewertung;
Nutzen des Platteneffektwerts als eine Punktschätzung für jede der Komponenten und Berechnen von Wahrscheinlichkeiten der geschätzten statistischen Parameter, wobei die statistischen Parameter den Mittelwert, die Standardabweichung und den Platteneffekt für die Komponenten einschließen;
Auswerten der Passung des Modells gegen eine Wahrscheinlichkeitstoleranz;
Auswerten einer Passung des nächsten Modells, falls das Modell nicht innerhalb der Wahrscheinlichkeitstoleranz ist; und
Anwenden des Modells mit der höchsten medianen Wahrscheinlichkeit über Parameter, falls keines der Vielzahl von Kandidatenmodellen die Wahrscheinlichkeitstoleranz erfüllt;
Konfigurieren eines Normalisators mit historischen Komponentendaten, um den Mittelwert und die Standardabweichung jeder der Komponenten einzustellen, um eine angepasste Mischung zu erzeugen;
Konfigurieren eines Klassifikators mit der angepassten Mischung, um unbekannte Abtastwerte zu klassifizieren, das Konfigurieren des Klassifikators umfassend:

Gewichten von Komponentendichten basierend auf der angepassten Mischung; und
Vergleichen der unbekannten Abtastwerte mit den wahrscheinlichsten Komponenten; und
Zuweisen eines Kopienzahlwertes, der der Komponente mit der höchsten Wahrscheinlichkeit entspricht, an die unbekannten Abtastwerte, falls das Verhältnis der Dichte der wahrscheinlichsten Komponente zu der Dichte der zweitwahrscheinlichsten Komponente, die an der Abtastwertposition ausgewertet wurden, über einer gewissen Höchstgrenze liegt, und die absolute Dichte der wahrscheinlichsten Komponente, die an der Abtastwertposition ausgewertet wurde, über einer Dichtehöchstgrenze liegt.

10. Recheneinrichtung nach Anspruch 9, wobei die Bewertungsfunktion als ein Produkt von vorherigen Dichten auf Mittelwerten von Mischungskomponenten, dem Platteneffekt und Gewichtungen der Komponenten aufgebaut ist.

11. Recheneinrichtung nach einem der Ansprüche 9 bis 10, wobei die Wahrscheinlichkeitstoleranz ferner umfasst, dass der Median der Wahrscheinlichkeiten größer als 0,1 ist und keine individuelle Wahrscheinlichkeit weniger als 0,001 beträgt.

12. Recheneinrichtung nach einem der Ansprüche 9 bis 11, wobei das Datendiagramm ein Diagramm umfasst, das Achsen aufweist, die die Dichte und das mediane log2-Verhältnis darstellen.

13. Recheneinrichtung nach Anspruch 12, wobei die medianen log2-Verhältniswerte den Median von log2-Verhältnissen von Intensitätsdaten zu einem Referenzwert über eine Vielzahl von Messungen einer genomischen Region hinweg umfassen.

14. Recheneinrichtung nach einem der Ansprüche 9 bis 13, wobei das Datendiagramm ein Diagramm umfasst, das Achsen aufweist, die die Dichte und eine beliebige Messung der zentralen Tendenz darstellen.

15. Recheneinrichtung nach einem der Ansprüche 9 bis 14, wobei das Anwenden des dichtebasierten Clusterings auf das Datendiagramm ferner umfasst:

    Erzeugen einer Kerneldichteschätzung aus dem Datendiagramm;
    Partitionieren des Datendiagramms in eine Vielzahl von Regionen basierend auf der Dichte lokaler Minima;
    Berechnen des Mittelwerts und der Standardabweichung der Punkte für jede Region;
    Zusammenführen von Werten innerhalb eines ersten spezifizierten Abstandswerts von einer anderen Region, falls die Anzahl von Beobachtungen unter einem ersten Schwellenwert ist;
    Entfernen von Regionen außerhalb des ersten spezifizierten Abstandswerts von einer beliebigen anderen Region;
    Berechnen des Mittelwerts, der Standardabweichung und der Proportion von Datenpunkten in jeder Region; und
    Erzeugen einer Vielzahl von vereinfachten Kandidatenmodellen, umfassend:

        Zusammenführen von Werten innerhalb eines zweiten spezifizierten Abstandswerts von einer anderen Region;
        Entfernen von Werten außerhalb des zweiten spezifizierten Abstandswerts von einer beliebigen anderen Region, falls die Anzahl der Beobachtungen unter einem Schwellenwert ist; und
        Berechnen des Mittelwerts, der Standardabweichung und der Proportion der Datenpunkte.

**Revendications**

1. Procédé mis en oeuvre par ordinateur pour le génotypage de variantes de nombre de copies comprenant :

    la réception de données de fluorescence pour un graphe de données à partir d'un microréseau comportant des sondes d'acide nucléique ;
    l'application d'un regroupement basé sur la densité au graphe de données pour générer une pluralité de modèles candidats à partir de composants gaussiens ;
    la sélection d'un modèle optimal à partir de la pluralité de modèles candidats, la sélection du modèle optimal comprenant :

        l'application d'un modèle à partir de la pluralité de modèles candidats, et d'une fonction de notation aux composants, pour générer une note de composant ;
        la sélection d'une valeur d'effet de plaque ;
        la sélection d'une étiquette de composant pour chaque composant sur la base de la note de composant ;
        l'utilisation de la valeur d'effet de plaque en tant qu'estimation de point pour chacun des composants et le calcul des probabilités des paramètres statistiques estimés du composant, les paramètres statistiques comportant la moyenne, l'écart type et la valeur d'effet de plaque pour les composants ;
l'évaluation de l'ajustement du modèle contre une tolérance de probabilité ;
l'évaluation d'un modèle suivant, si le modèle n'est pas dans la tolérance de probabilité ; et
l'application du modèle avec la probabilité médiane la plus élevée sur les paramètres, si aucun modèle de la pluralité de modèles candidats ne satisfait la tolérance de probabilité ;
la configuration d'un normalisateur avec des données de composante historiques pour ajuster la moyenne et l'écart type de chacun des composants afin de générer un mélange ajusté ;
la configuration d'un classificateur avec le mélange ajusté pour classer des échantillons inconnus, la configuration du classificateur comprenant :

la pondération de densités de composants sur la base du mélange ajusté ; et
la comparaison des échantillons inconnus aux composantes les plus probables ; et
l'attribution d'un appel de numéro de copie correspondant au composant avec la probabilité la plus élevée aux échantillons inconnus, si le rapport de la densité de la composante la plus probable à la densité du second composant le plus probable, évalué à la position d'échantillon, est supérieur à une certaine coupure, et la densité absolue de la composante la plus probable, évaluée à la position d'échantillon, est supérieure à une coupure de densité.

2. Procédé selon la revendication 1, dans lequel la fonction de notation est construite en tant que produit de densités antérieures sur des moyens de composants de mélange, l'effet de plaque et les pondérations des composants.

3. Procédé selon l'une quelconque des revendications 1 ou 2, dans lequel la tolérance de probabilité comprend en outre la médiane des probabilités qui est supérieure à 0,1 et aucune probabilité individuelle n'est inférieure à 0,001.

4. Procédé selon l'une quelconque des revendications 1 à 3, dans lequel le graphique de données comprend un graphique ayant des axes représentant une densité et un rapport médian log2.

5. Procédé selon l'une quelconque des revendications 1 à 4, dans lequel les valeurs de rapport médian log2 comprennent les rapports médians log2 de données d'intensité à une valeur de référence, à travers une pluralité de mesures d'une région génomique.

6. Procédé selon la revendication 5, dans lequel les données d'intensité comportent des mesures d'intensité fluorescentes provenant d'un microréseau.

7. Procédé selon l'une quelconque des revendications 1 à 6, dans lequel le graphe de données comprend un graphique ayant des axes représentant la densité et toute mesure de tendance centrale.

8. Procédé selon l'une quelconque des revendications 1 à 7, dans lequel l'application d'un regroupement basé sur la densité au graphe de données comprend en outre :

la génération d'une estimation de densité de noyau à partir du graphe de données ;
la partition du graphe de données en une pluralité de régions sur la base de la mini-valeur locale de densité ;
le calcul de la moyenne et l'écart type de points pour chaque région ;
la fusion de valeurs dans une première valeur de distance spécifiée d'une autre région si le nombre d'observations est inférieur à une première valeur seuil ;
l'élimination des régions à l'extérieur de la première valeur de distance spécifiée de toute autre région ;
le calcul de la moyenne, l'écart type et la proportion de points de données dans chaque région ; et
la génération d'une pluralité de modèles candidats simplifiés comprenant :

des valeurs de fusion dans une seconde valeur de distance spécifiée à partir d'une autre région ;
l'élimination des valeurs en dehors de la seconde valeur de distance spécifiée de toute autre région, si le nombre d'observations est inférieur à une valeur seuil ; et
le calcul de la moyenne, l'écart type et la proportion des points de données.

9. Appareil informatique pour génotyper des variantes de numéro de copie, l'appareil informatique comprenant :

un processeur ; et
une mémoire stockant des instructions qui, lorsqu'elles sont exécutées par le processeur, configurent l'appareil pour :

recevoir des données de fluorescence pour un graphe de données à partir d'un microréseau comportant des sondes d'acide nucléique ;

appliquer un regroupement basé sur la densité au graphe de données pour générer une pluralité de modèles candidats à partir de composants gaussiens ;

sélectionner un modèle optimal parmi la pluralité de modèles candidats, la sélection du modèle optimal comprenant :

l'application d'un modèle parmi la pluralité de modèles candidats et d'une fonction de notation, aux composants, pour générer une note de composant ;

la sélection d'une valeur d'effet de plaque ;

la sélection d'une étiquette de composant pour chaque composant sur la base de la note de composant ;

l'utilisation de la valeur d'effet de plaque en tant qu'estimation de point pour chacun des composants et le calcul des probabilités des paramètres statistiques estimés du composant, les paramètres statistiques comportant la moyenne, l'écart type et la valeur d'effet de plaque pour les composants ;

l'évaluation de l'ajustement du modèle contre une tolérance de probabilité ;

l'évaluation d'un modèle suivant, si le modèle n'est pas dans la tolérance de probabilité ; et

l'application du modèle avec la probabilité médiane la plus élevée sur les paramètres, si aucun de la pluralité de modèles candidats ne satisfait la tolérance de probabilité ;

la configuration d'un normalisateur avec des données de composante historiques pour ajuster la moyenne et l'écart type de chacun des composants pour générer un mélange ajusté ;

la configuration d'un classificateur avec le mélange ajusté pour classer des échantillons inconnus, la configuration du classificateur comprenant :

la pondération de densités de composants sur la base du mélange ajusté ; et

la comparaison des échantillons inconnus aux composants les plus probables ; et

l'attribution d'un appel de numéro de copie correspondant au composant avec la probabilité la plus élevée aux échantillons inconnus, si le rapport de la densité de la composante la plus probable à la densité du second composant le plus probable, évalué à la position d'échantillon, est supérieur à une certaine coupure, et la densité absolue de la composante la plus probable, évaluée à la position d'échantillon, est supérieure à une coupure de densité.

10. Appareil informatique selon la revendication 9, dans lequel la fonction de notation est construite en tant que produit de densités antérieures sur des moyens de composants de mélange, l'effet de plaque et les pondérations des composants.

11. Appareil informatique selon l'une quelconque des revendications 9 à 10, dans lequel la tolérance de probabilité comprend en outre la médiane des probabilités qui est supérieure à 0,1 et aucune probabilité individuelle n'est inférieure à 0,001.

12. Appareil informatique selon l'une quelconque des revendications 9 à 11, dans lequel le graphe de données comprend un graphique ayant des axes représentant une densité et un rapport médian log2

13. Appareil informatique selon la revendication 12, dans lequel les valeurs de rapport médian log2 comprennent la médiane de rapports log2 de données d'intensité à une valeur de référence, à travers une pluralité de mesures d'une région génomique.

14. Appareil informatique selon l'une quelconque des revendications 9 à 13, dans lequel le graphe de données comprend un graphique ayant des axes représentant la densité et toute mesure de tendance centrale.

15. Appareil informatique selon l'une quelconque des revendications 9 à 14, dans lequel l'application d'un regroupement basé sur la densité au graphe de données comprend en outre :

la génération d'une estimation de densité de noyau à partir du graphe de données ;

la partition du graphe de données en une pluralité de régions sur la base de la mini-valeur locale de densité ;

le calcul de la moyenne et l'écart type de points pour chaque région ;

la fusion de valeurs dans une première valeur de distance spécifiée d'une autre région si le nombre d'obser-

NO

vations est inférieur à une première valeur seuil ;

l'élimination des régions à l'extérieur de la première valeur de distance spécifiée de toute autre région ;

le calcul de la moyenne, l'écart type et la proportion de points de données dans chaque région ; et

la génération d'une pluralité de modèles candidats simplifiés comprenant :

des valeurs de fusion dans une seconde valeur de distance spécifiée à partir d'une autre région ;

l'élimination des valeurs en dehors de la seconde valeur de distance spécifiée de toute autre région, si le nombre d'observations est inférieur à une valeur seuil ; et

le calcul de la moyenne, l'écart type et la proportion des points de données.

100

ANALYTIC
COMPUTER
SYSTEM 102

SENSORS
106

MICROARRAY
108

REACTION PLATE 104

**FIG. 1**

**FIG. 2**

300

APPLYING A MODEL FROM THE PLURALITY OF CANDIDATE MODELS, AND A SCORING FUNCTION TO THE COMPONENTS, TO GENERATE A COMPONENT SCORE 302

SELECT BEST-FIT MODEL FROM PLURALITY OF CANDIDATE MODELS 304

CONFIGURE A NORMALIZER WITH HISTORICAL COMPONENT DATA TO ADJUST THE MEAN AND STANDARD DEVIATION OF EACH OF THE COMPONENTS TO GENERATE AN ADJUSTED MIXTURE 306

CONFIGURING A CLASSIFIER WITH THE ADJUSTED MIXTURE TO CLASSIFY UNKNOWN SAMPLES 308

WEIGHT COMPONENT DENSITIES BASED ON THE ADJUSTED MIXTURE 310

COMPARE THE UNKNOWN SAMPLES TO THE MOST PROBABLE COMPONENTS 312

DENSITY RATIO ABOVE CUTOFF 314

NO

NO CALL ASSIGNED 318

YES

DENSITY ABOVE DENSITY CUTOFF 316

NO

YES

ASSIGN A LABEL FOR THE COMPONENT WITH THE HIGHEST PROBABILITY TO THE UNKNOWN SAMPLES 320

FIG. 3

400

```
┌─────────────────────────────────────────────┐
│ APPLY A MODEL FROM THE PLURALITY OF CANDIDATE │
│ MODELS, AND A SCORING FUNCTION TO THE COMPONENTS│
│    TO GENERATE A COMPONENT SCORE 402          │
└─────────────────────────────────────────────┘
                      │
                      ▼
┌─────────────────────────────────────────────┐
│       SELECT A PLATE EFFECT VALUE 404         │
└─────────────────────────────────────────────┘
                      │
                      ▼
┌─────────────────────────────────────────────┐
│ SELECT A COMPONENT LABEL FOR EACH COMPONENT BASED│
│       ON THE COMPONENT SCORE 406              │
└─────────────────────────────────────────────┘
                      │
                      ▼
┌─────────────────────────────────────────────┐
│ UTILIZE THE PLATE EFFECT VALUE AS A POINT ESTIMATE FOR│
│ EACH OF THE COMPONENTS AND CALCULATE PROBABILITIES│
│ OF THE COMPONENT'S ESTIMATED STATISTICAL PARAMETERS│
│                    408                        │
└─────────────────────────────────────────────┘
                      │
                      ▼
┌─────────────────────────────────────────────┐
│     EVALUATE THE MODEL'S FIT AGAINST A PROBABILITY│
│              TOLERANCE 410                     │
└─────────────────────────────────────────────┘
                      │
                      ▼
┌─────────────────────────────────────────────┐
│ EVALUATE A NEXT MODEL IF THE MODEL IS NOT WITHIN THE│
│        PROBABILITY TOLERANCE 412              │
└─────────────────────────────────────────────┘
                      │
                      ▼
┌─────────────────────────────────────────────┐
│ APPLY THE MODEL WITH THE HIGHEST MEDIAN PROBABILITY│
│    OVER PARAMETERS IF NO ONE OF THE PLURALITY OF│
│ CANDIDATE MODELS MEETS THE PROBABILITY TOLERANCE│
│                    414                        │
└─────────────────────────────────────────────┘
```

**FIG. 4**

**FIG. 5**

FIG. 6

FIG. 7

**FIG. 8**

**FIG. 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 778358 B2